(19) Europäisches Patentamt
European Patent Office
Office européen des brevets

(11) Numéro de publication: **0 164 290**
**B1**

(12) # FASCICULE DE BREVET EUROPEEN

(45) Date de publication du fascicule du brevet: **06.09.89**

(51) Int. Cl.⁴: **C 07 D 489/00**

(21) Numéro de dépôt: **85400996.6**

(22) Date de dépôt: **21.05.85**

(54) **Procédé de déalkylation d'alcaloides et intermédiaires.**

(30) Priorité: **25.05.84 FR 8408273**

(43) Date de publication de la demande: **11.12.85 Bulletin 85/50**

(45) Mention de la délivrance du brevet: **06.09.89 Bulletin 89/36**

(84) Etats contractants désignés: **AT BE CH DE FR GB IT LI LU NL SE**

(56) Documents cités:
**FR-A- 2 395 269**
**FR-A- 2 515 184**
**US-A- 4 141 807**
**US-A- 4 390 699**

**JOURNAL OF MEDICINAL CHEMISTRY, vol. 15, no. 2, février 1972, pages 208-210, Columbus, Ohio, US; M.M. ABDEL-MONEM et al.: "N-demethylation of morphine and structurally related compounds with chloroformate esters"**

**Le dossier contient des informations techniques présentées postérieurement au dépôt de la demande et ne figurant pas dans le présent fascicule.**

(73) Titulaire: **SANOFI S.A., 40, Avenue George V, F-75008 Paris (FR)**

(72) Inventeur: **Hoff, Christian Mazet de l'Escoraille, Quartier de la Cabanette, F-30150 Saint Laurent des Arbres (FR)**
Inventeur: **Vergely, Christian, H.L.M. La Graze, F-30390 Aramon (FR)**

(74) Mandataire: **Gillard, Marie-Louise et al, Cabinet Beau de Loménie 55, Rue d'Amsterdam, F-75008 Paris (FR)**

**EP 0 164 290 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La thébaïne, alcaloïde extrait du pavot ou de l'opium ne présente pas de propriétés utilisables en thérapeutique par suite de sa toxicité extrêmement élevée.

Par contre, par une série de réactions chimiques, il est possible de transformer la thébaïne en diverses substances possédant d'intéressantes propriétés thérapeutiques, principalement des propriétés analgésiques et/ou antagonistes des opiacés.

Parmi ces substances, on peut citer les composés de formule:

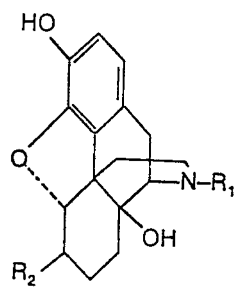

à savoir:

| | | |
|---|---|---|
| la naloxone | $R_1 = -CH_2CH = CH_2$ | $R_2 = 0$ |
| la naltrexone | $R_1 = -CH_2-$ 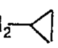 | $R_2 = 0$ |
| la nalbuphine | $R_1 = -CH_2-$ 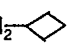 | $R_2 = -OH$ |
| le nalméfène | $R_1 = -CH_2-$ 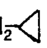 | $R_2 =$ $CH_2$ |

qui sont tous des dérivés de 14-hydroxy-morphinane.

Ces composés comportant sur l'atome d'azote un substituant différent de celui porté par la thébaïne (groupe méthyle), il apparaît que la déméthylation de l'amine tertiaire de la thébaïne constitue une étape obligatoire de toutes ces synthèses.

La déméthylation de la thébaïne, avant toute autre transformation, ne peut être réalisée qu'avec de faibles rendements dus à l'instabilité du produit de départ et surtout à celle du produit déméthylé obtenu. Aussi, ce procédé est inutilisable industriellement.

La thébaïne peut par contre être transformée, par des procédés connus, en dérivés 14-hydroxylés:

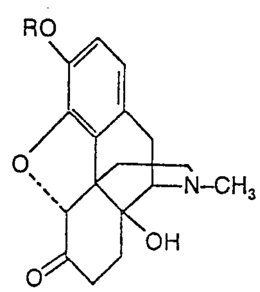

désignés sous le nom de 14-hydroxy-dihydrocodéïnone ou encore oxycodone lorsque $R = CH_3$ et sous le nom de 14-hydroxy-dihydromorphinone ou oxymorphone lorsque R = H. De tels procédés sont décrits notamment dans: The Chemistry of the morphine alkaloids par K. W. Bentley Editeur Oxford At the Clarendon Press (1954) et le brevet FR 2 439 201.

C'est à partir de ces molécules que sera effectuée la déméthylation de l'azote. Toutefois, pour éviter des réactions indésirables, il est nécessaire que le ou les hydroxyles présents dans la molécule soient protégés. Le plus souvent, on formera l'ester correspondant par action de l'anhydride acétique. Le groupe 14-acétoxy provoque alors un encombrement stérique important qui limite considérablement les possibilités de déméthylation.

C'est pour cette raison que différents réactifs proposés pour la déméthylation d'alcaloïdes, notamment en série morphinique, ne donnent avec les 14-hydroxymorphinanes que des rendements médiocres nécessitant des séparations laborieuses pour isoler le composé déméthylé.

C'est le cas particulier du chloroformiate de phényle, du chloroformiate de trichloroéthyle et du chloroformiate d'alpha-chloroéthyle.

Dans le cas des 14-hydroxymorphinanes, seuls deux réactifs ont été utilisés à ce jour. Il s'agit tout d'abord du bromure de cyanogène mentionné par J. VON BRAUN Berichte 47, 2312, (1914) en série morphinique et utilisé par M. LEWENSTEIN Brevet US N° 3 254 088 en série 14-hydroxymorphinique.

Ce réactif fournit des rendements de l'ordre de 70% en produit déméthylé. Il est extrêmement toxique et son emploi industriel exige de très grandes précautions d'utilisation.

Il s'agit ensuite du chloroformiate de vinyle proposé par R. OLOFSON (brevets US n° 3 905 981 et 4 141 897). Avec ce réactif, le rendement en produit déméthylé varie de 70 à 85%. Ce réactif présente au plan industriel deux inconvénients importants: son instabilité conduisant à des rendements variables d'une part, sa préparation délicate entraînant un coût élevé d'autre part.

Selon la présente invention, il a été trouvé que d'une façon surprenante, on peut procéder à la déméthylation de l'azote des 14-hydroxymorphinanes en utilisant comme réactif le chloroformiate d'éthyle.

Ce réactif a déjà été utilisé en tant qu'agent de N-déméthylation d'alcaloïdes. Dès 1921, J. GADAMER et F. KNOCH, Arch. Phar. 259, 135–158, (1921) étudiant l'action du chloroformiate d'éthyle sur des alcaloïdes N-méthylés concluaient que la morphine, la codéine et l'héroïne n'étaient pas clivés.

Plus récemment, E. JUCKER et A. LINDENMANN (brevet suisse n° 442 318) et G. KRAISS et K. NADOR (Tetrahedron Letters 1, 57–58, 1971) ont mentionné l'utilisation du chloroformiate d'éthyle comme agent de déméthylation de l'azote de dérivés de la famille du tropane. Les rendements obtenus sont assez peu élevés.

M. ABDEL-MONEN et P. PORTOGHESE, J. Med. Chem. 15 (2), 208–210, (1972) ont déméthylé la morphine et la codéine par action du chloroformiate d'éthyle avec des rendements de l'ordre de 40%.

De même, K. RICE et E. MAY, J. Heterocyclic Chem. 14, 665, (1977) arrivent à la même conclusion.

Enfin, M. SCHWARTZ et R. WALLACE, J. Med. Chem. 24, 1525–1528, (1981) font agir le chloroformiate d'éthyle sur la codéine pour former la N-éthoxycarbonyl-codéine qui sera soumise à toute une série de réactions en vue de sa transformation en dérivé 14-hydroxymorphinane, lequel sera hydrolysé à ce moment en dérivé N-nor correspondant.

Compte tenu des difficultés connues pour la déméthylation des 14-hydroxymorphinanes exposées précédemment, aucun de ces documents ne pouvaient laisser espérer des résultats favorables en utilisant le chloroformiate d'éthyle pour déméthyler l'oxycodone ou l'oxymorphone.

Pourtant, d'une façon tout à fait inattendue, les résultats obtenus avec le chloroformiate d'éthyle montrent la supériorité de ce réactif sur ceux utilisés à ce jour.

Selon le procédé, objet de la présente invention, on forme le dérivé N-éthoxycarbonyl par action du chloroformiate d'éthyle en excès sur le dérivé à déméthyler en présence de carbonate de potassium et au sein d'un solvant de préférence le dichlorométhane ou le chloroforme au reflux. Les dérivés N-éthoxycarbonyle des 14-acétoxy-dihydronor-codéinone et nor-morphinone sont nouveaux et constituent des intermédiaires clés du présent procédé; ils font à ce titre partie de l'invention. Le procédé de l'invention est illustré par le schéma ci-après:

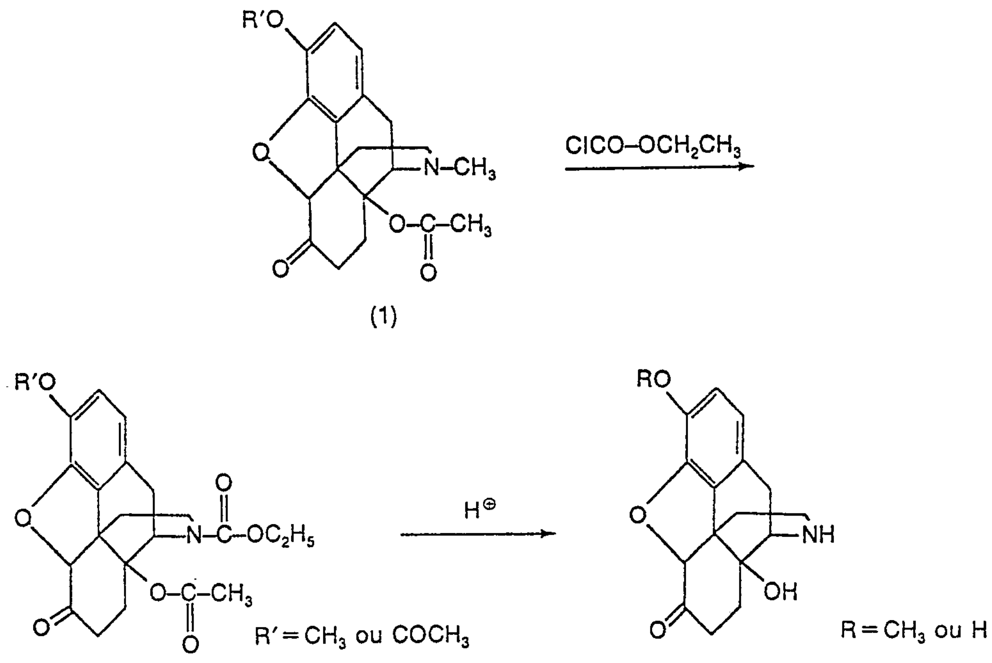


(1)

(2) R' = $CH_3$ ou $COCH_3$

(3) R = $CH_3$ ou H

L'hydrolyse du carbamate est effectuée en milieu acide fort, particulièrement par l'acide sulfurique de concentration comprise entre 5 et 10 N. De préférence, on utilise un mélange acide acétique-acide sulfurique au reflux, mélange qui produit moins de dégradations que l'acide sulfurique seul. L'hydrolyse du carbamate s'accompagne de la saponification du ou des esters acétiques présents dans la molécule.

L'exemple suivant permettra de mieux comprendre l'invention.

A) N-éthoxycarbonyl 14 – acétoxydihydronorcodéinone.
(2) R' = $CH_3$

On chauffe au reflux pendant 21 heures, le mélange de 28 g d'acétyl-14 oxycodone [(1) R' = $CH_3$], 12,5 g de carbonate de potassium et 45 ml de chloroformiate d'éthyle dans 30 ml de chloroforme.

Après refroidissement, on lave le mélange réactionnel avec 150 ml d'eau. On sépare la phase aqueuse que l'on réextrait 2 fois avec 50 ml de chloroforme.

On réunit les extraits chloroformiques et on évapore à siccité. On obtient une huile brunâtre utilisée telle quelle pour l'opération suivante.

Eventuellement, on peut préparer un échantillon analytique du produit en dissolvant l'huile dans le méthanol à reflux. Par refroidissement, il se sépare des cristaux qu'on essore, lave avec de l'éther isopropylique.

Après séchage, F: 146–147 °C

Chromatographie en couche mince: 1 seule tache

(Benzène-Acétone, 70–30 vol/vol).

Analyse élémentaire:

| | | | |
|---|---|---|---|
| Calculée: | C: 63,60 | H: 6,07 | N: 3,37 |
| Trouvée: | C: 63,61 | H: 6,04 | N: 3,28 |

B) Noroxycodone (3) R = $CH_3$

On reprend l'huile obtenue ci-dessus dans 30 ml d'acide acétique puis on ajoute le mélange de 20 ml d'acide sulfurique 36 N et 140 ml d'eau. On chauffe au reflux pendant 21 heures. Après refroidissement, on amène le pH du mélange réactionnel à 11 par addition d'une solution de soude à 30% en refroidissant pour maintenir la température du mélange à 25 °C environ. On ajoute 100 ml de chloroforme, refroidit à 0 °C et filtre en présence d'adjuvant de filtration. On lave le gâteau 2 fois avec 100 ml de chloroforme. On décante le chloroforme et extrait la phase aqueuse 3 fois avec 100 ml de chloroforme. On réunit les extraits chloroformiques et évapore à siccité sous vide.

Le résidu solide est repris dans le mélange de 15 ml d'acide acétique et 150 ml d'eau. On dissout le solide par chauffage à 35–40 °C puis on alcalinise la solution par addition d'ammoniaque en refroidissant à 20 °C. On essore le solide, lave avec de l'eau jusqu'à neutralité et sèche à l'étuve à 80 °C.

On obtient 21,6 g de noroxycodone.

F: 160 °C (avec décomposition).

Rendement 92% pour l'ensemble des 2 étapes.

Ce produit est en tout point identique avec un échantillon authentique de noroxycodone.

Le procédé selon l'invention donne donc des résultats particulièrement avantageux.

Afin de montrer la supériorité de ce procédé, on a répété l'opération décrite au paragraphe A) de l'exemple, c'est-à-dire, la formation du carbamate en faisant varier la nature du chloroformiate utilisé.

Dans chaque cas, on a déterminé, par extraction par l'acide chlorhydrique dilué, la quantité de produit de départ non transformé en carbamate.

Les résultats obtenus figurent dans le tableau 1 ci-après:

Tableau 1

| Chloroformiate utilisé | Oxycodone non transformé en carbamate % |
|---|---|
| Chloroformiate d'éthyle $ClCOOC_2H_5$ | 4 |
| Chloroformiate de trichloroéthyle $ClCOOCH_2CCl_3$ | 40 |
| Chloroformiate de alpha-chloroéthyle<br>$ClCOOCH-CH_3$<br>Cl | 26 |
| Chloroformiate d'allyle $ClCOOCH_2CH=CH_2$ | 30 |
| Chloroformiate de vinyle $ClCOOCH=CH_2$ | 7 |
| Chloroformiate de méthyle $ClCOOCH_3$ | 13 |

Ces résultats montrent clairement que lors de la réaction d'un 14-hydroxymorphinane avec un chloroformiate pour former le carbamate correspondant, c'est le chloroformiate d'éthyle qui donne le meilleur taux de transformation.

Ce taux de transformation est suffisamment élevé pour ne pas nécessiter de purification du carbamate avant hydrolyse acide. Celle-ci conduit directement et avec un rendement élevé à un produit pur après une seule cristallisation.

De plus, le procédé selon l'invention présente les avantages suivants:

– utilisation d'un réactif, le chloroformiate d'éthyle, parfaitement stable, ce qui permet, entre autre, l'obtention de rendements reproductibles;

– utilisation d'un réactif moins toxique que ceux utilisés jusqu'à ce jour, à savoir le bromure de cyanogène et le chloroformiate de vinyle;

– utilisation d'un réactif peu coûteux du fait de sa facilité de préparation.

**Revendications**

1. Procédé de déalkylation des alcaloïdes 14-hydroxymorphinanes, caractérisé en ce qu'il consiste à:

1) faire réagir le dérivé 14-hydroxylé de formule

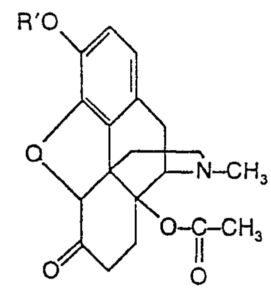

dans laquelle R' est $CH_3$ ou $COCH_3$ avec du chloroformiate d'éthyle en excès en présence de carbonate de potassium et au sein d'un solvant,

2) à soumettre le carbamate ainsi obtenu de formule:

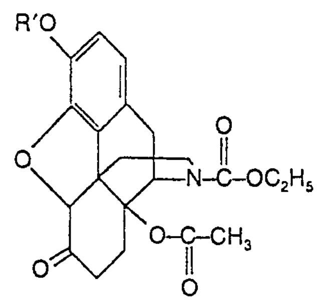

(2)

dans laquelle R' est $CH_3$ ou $COCH_3$ à un hydrolyse en milieu acide fort pour obtenir le dérivé déalkylé correspondant de formule:

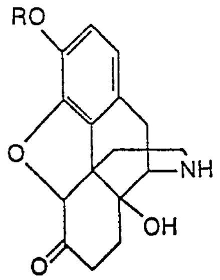

(3)

dans laquelle R est $CH_3$ ou l'hydrogène.

2. Procédé selon la revendication 1, caractérisé en ce que la première étape est réalisée dans du dichlorométhane ou du chloroforme au reflux.

3. Procédé selon l'une des revendications 1 ou 2, caractérisé en ce que l'hydrolyse est effectuée à l'aide d'acide sulfurique.

4. Procédé selon l'une des revendications 1 ou 2, caractérisé en ce que l'hydrolyse est effectuée à l'aide d'un mélange acide acétique- acide sulfurique au reflux.

5. A titre de produits intermédiaires utiles dans le procédé selon l'une quelconque des revendications 1 à 4, les dérivés N-éthoxycarbonyle des 14-acétoxy-dihydro-norcodéinone et normorphinone de formule:

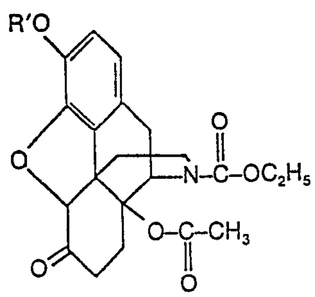

(2)

dans laquelle R' est $CH_3$ ou $COCH_3$.

**Claims**

1. A process for the dealkylation of 14-hydroxymorphinan alkaloids, characterized in that it consists in:

1) reacting the 14-hydroxy derivative of the formula:

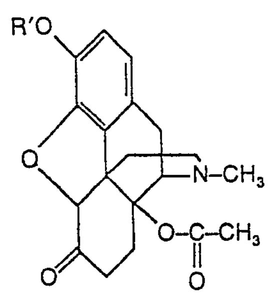

in which R' is $CH_3$ or $COCH_3$, with excess ethyl chloroformate, in the presence of potassium carbonate and in a solvent,

2) subjecting the resulting carbamate of the formula:

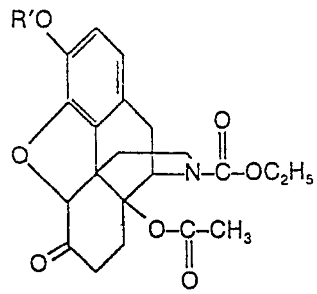

(2)

in which R' is $CH_3$ or $COCH_3$, to hydrolysis in a strong acid medium to give the corresponding dealkylated derivative of the formula:

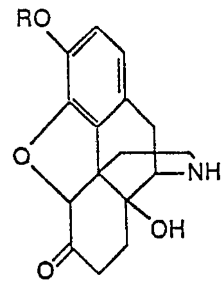

(3)

in which R is $CH_3$ or hydrogen.

2. The process according to claim 1, characterized in that the first step is carried out in methylene chloride or chloroform under reflux.

3. The process according to one of claims 1 or 2, characterized in that the hydrolysis is carried out with sulfuric acid.

4. The process according to one of claims 1 or 2, characterized in that the hydrolysis is carried out with an acetic acid/sulfuric acid mixture under reflux.

5. As intermediates useful in the process according to any one of claims 1 to 4, the N-ethoxycarbonyl derivatives of 14-acetoxydihydronorcodeinone and normorphinone of the formula:

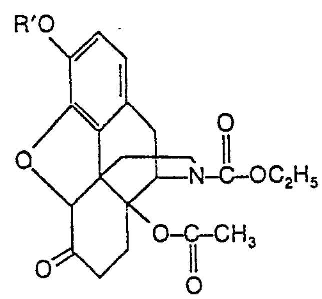

(2)

in which R' is $CH_3$ or $COCH_3$.

**Patentansprüche**

1. Verfahren zur Entalkylierung von 14-Hydroxymorphinanalkaloiden, dadurch gekennzeichnet, daß es darin besteht, daß:

1) das 14-Hydroxylderivat der Formel

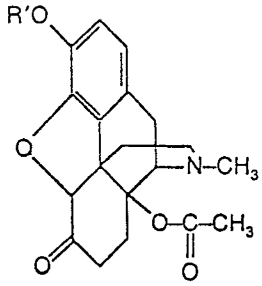

worin R′ für $CH_3$ oder $COCH_3$ steht, mit Ethylchlorformiat im Überschuß in Gegenwart von Kaliumkarbonat in einem Lösungsmittel reagieren gelassen wird,

2) das so erhaltene Karbamat der Formel:

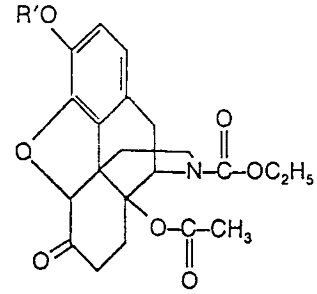

(2)

worin R′ für $CH_3$ oder $COCH_3$ steht, einer Hydrolyse in stark saurem Milieu unterzogen wird, um das entsprechende entalkylierte Derivat der Formel

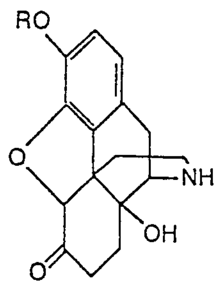

(3)

worin R für $CH_3$ oder Wasserstoff steht, zu erhalten.

2. Verfahren nach Anspruch 1, dadurch gekennzeichnet, daß die erste Stufe in Dichlormethan oder Chloroform unter Rückfluß durchgeführt wird.

3. Verfahren nach einem der Ansprüche 1 oder 2, dadurch gekennzeichnet, daß die Hydrolyse mit Hilfe von Schwefelsäure durchgeführt wird.

4. Verfahren nach einem der Ansprüche 1 oder 2, dadurch gekennzeichnet, daß die Hydrolyse mit Hilfe einer Mischung aus Essigsäure-Schwefelsäure unter Rückfluß durchgeführt wird.

5. Als für das Verfahren nach einem der Ansprüche 1 bis 4 nützliche Zwischenprodukte die N-Ethoxycarbonylderivate von 14-Acetoxy-dihydronorcodeinon und -normorphinon der Formel:

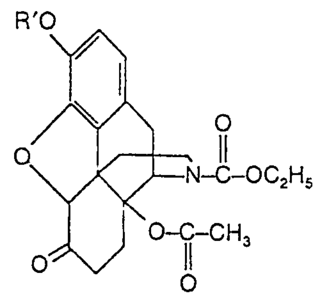

(2)

worin R′ für $CH_3$ oder $COCH_3$ steht.